Europäisches Patentamt
European Patent Office
Office européen des brevets

(11) Publication number: **0 034 010**
**A1**

# EUROPEAN PATENT APPLICATION

(21) Application number: 81300330.8

(22) Date of filing: 26.01.81

(51) Int. Cl.³: **C 07 C 159/00**, C 07 C 133/08, C 07 D 285/12, A 01 N 47/34, A 01 N 47/42, A 01 N 47/36

(30) Priority: 06.02.80 US 118936

(43) Date of publication of application: 19.08.81 Bulletin 81/33

(84) Designated Contracting States: FR GB IT

(71) Applicant: **Gulf Oil Corporation, P. O. Box 1166, Pittsburgh Pennsylvania 15230 (US)**

(72) Inventor: **Rutter, Jerry Lynn, 6128 Norwood, Mentor Ohio 44060 (US)**
Inventor: **Kirkpatrick, Joel Lee, 17 Canal Run East, Washington Crossing Pennsylvania 18977 (US)**
Inventor: **Gibbs, Charles Gaylord, 1901 West 72nd Terrace, Shawnee Mission Kansas 66208 (US)**

(74) Representative: **Huskisson, Frank Mackie et al, Fitzpatricks 48 St. Vincent Street, Glasgow, G2 5TT Scotland (GB)**

(54) **Substituted thiosemicarbazones and use as plant growth regulants.**

(57) A method of regulating the growth of plants including combating unwanted vegetation comprises applying to the plants, either on seed, the soil or directly on the plants an effective amount of a compound having the general structural formula

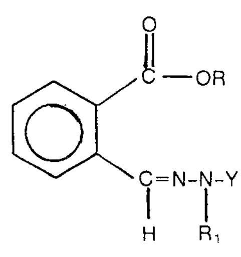
in which Y is
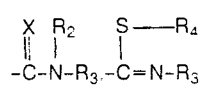

or
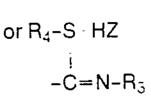
and X is O or S

R is H, $C_1$ to $C_4$ alkyl or an agriculturally acceptable cation
$R_1$ is H or $C_1$ to $C_4$ alkyl or hydroxyalkyl
$R_2$ is H or $C_1$ to $C_3$ alkyl
$R_3$ is H, $C_1$ to $C_8$ alkyl, cycloalkyl or alkenyl, adamantyl, naphthyl, 1,3,4-thiadiazol-2-yl, phenyl or mono- or disubstituted phenyl in which the substituents are selected from $C_1$ to $C_4$ alkyl, $C_1$ to $C_4$ alkoxy, benzyloxy, chloro, bromo, fluoro, iodo, cyano or trifluoromethyl, Z is halogen and $R_4$ is $C_1$ to $C_3$ alkyl, allyl, benzyl of carboethoxymethyl.

EP 0 034 010 A1

# Substituted thiosemicarbazones and use as plant growth regulants.

## Description of the Invention

Many chemical substances have been found to have growth regulating effects on plants. Change of shape of plants, crinkling and folding of leaves, stunting of growth, defoliation, stimulation of root growth on cuttings and other effects are frequently observed. It is usually necessary to apply growth regulating substances to plants at comparatively low rates in order to have the growth regulating effects clearly evident. At higher rates a selective kill of vegetation is usually obtained, as some species of plants are affected more than others. Growth regulants have, in fact been used more as selective herbicides than for other purposes because in many instances the growth regulating effects exhibited by these substances have no other practical utility. However, a class of growth regulator compounds has now been discovered that produces a number of useful effects, differing from one plant species to another. For example, some of these compounds promote tillering (branching at the base of the plant) in oats and other grains and taller growth and increase of fruit set in tomatoes and certain other plants.

Briefly, the class of growth regulator compounds has the general structural formula

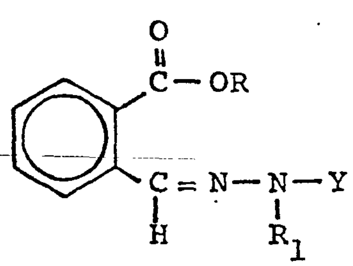 in which Y is 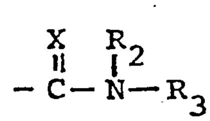, 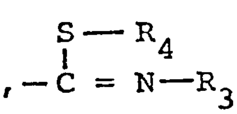

or 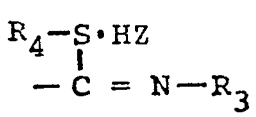 and X is O or S

R is H, $C_1$ to $C_4$ alkyl or an agriculturally acceptable cation

$R_1$ is H or $C_1$ to $C_4$ alkyl or hydroxyalkyl

$R_2$ is H or $C_1$ to $C_3$ alkyl

$R_3$ is H, $C_1$ to $C_8$ alkyl, cycloalkyl or alkenyl, adamantyl, naphthyl, 1,3,4-thiadiazol-2-yl, phenyl or mono- or disubstituted phenyl in which the substituents are selected from $C_1$ to $C_4$ alkyl, $C_1$ to $C_4$ alkoxy, benzyloxy, chloro, bromo, fluoro, iodo, cyano or trifluoromethyl, Z is halogen and $R_4$ is $C_1$ to $C_3$ alkyl, allyl, benzyl or carboethoxymethyl.

## Synthesis of the Growth Regulators

The novel class of growth regulators may be synthesized by means of the following illustrative procedures.

### Preparation of 2-Carbomethoxybenzaldehyde: Ref: J. Chem. Soc. (C), p. 1818(1979).

To a solution of 24.8g (0.165 mole) of phthalaldehydic acid in 450 ml of acetone was added 65 g (0.47 mole) of potassium carbonate and 29.8g(0.21 mole) of methyl iodide. The mixture was refluxed overnight, cooled, and filtered. The acetone was removed by evaporation, the residue dissolved in ether, washed with water, dried and filtered. Evaporation gave 22g of the desired product as an oil. The IR and NMR spectra were consistent with the proposed structure.

### Preparation of 1-(2'-Carboxybenzylidene)-2-methyl-4-phenyl-3-thiosemicarbazide

To a suspension of 3.6g (0.02 mole) of 2-methyl-4-phenyl-3-thiosemicarbazide in 50 ml of glacial acetic acid was added 3.0g (0.02 mole) of phthalaldehydic acid. After stirring at room temperature for 16 hrs. water was added and the product collected by filtration to give 4.2g mp 154-155°C.

Preparation of Methyl [1-(2'-Carboxybenzylidene)-2-methyl-4-(4'-fluorophenyl)]-isothiosemicarbazonium iodide

To a solution of 2.5g of 1-(2'-Carboxybenzylidene)-2-methyl-4-(4'-fluorophenyl)-3-thiosemicarbazide in 10 ml of dimethylformamide was added 5 ml of methyl iodide. After stirring at room temperature for 16 hrs, ethyl acetate was added and the resulting solid collected. Yield 1.2g, mp: 120-123°(dec). If desired, the salts so obtained may be neutralized to the corresponding free bases by treatment with aqueous base.

Compounds which have been made by essentially the procedures illustrated above are listed in the following table.

Compounds of the formula 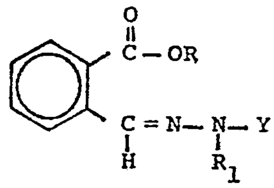

| Comp'd. No. | R | $R_1$ | Y | $R_2$ | $R_3$ | X | Z | m.p.°C |
|---|---|---|---|---|---|---|---|---|
| 2458 | $CH_3$ | $CH_3$ | $-\overset{X}{\overset{\|}{C}}-\overset{R_2}{\overset{\|}{N}}-R_3$ | H | phenyl | S | - | 138-140 |
| 2556 | " | " | " | " | 4-chlorophenyl | " | - | 172-173 |
| 2557 | " | " | " | " | 3-fluorophenyl | " | - | 123-125 |
| 2558 | " | " | " | " | 4-fluorophenyl | " | - | 124-126 |
| 2924 | " | " | " | " | 3-chlorophenyl | " | - | 138-139 |
| 2925 | " | H | " | " | 3-chloro-4-methylphenyl | " | - | 190-192 |
| 2926 | " | " | " | " | 2-chlorophenyl | " | - | 179-181 |
| 2927 | " | " | " | " | 3-chlorophenyl | " | - | 168-170 |
| 2928 | " | " | " | " | 4-nitrophenyl | " | - | 199-200 |
| 2930 | " | $CH_3$ | " | " | 4-methoxyphenyl | " | - | 110-111 |
| 2931 | " | " | " | " | 2-methylphenyl | " | - | 144-149 |
| 2932 | " | H | " | " | 3-trifluoromethylphenyl | " | - | 155-158 |
| 2933 | " | $CH_3$ | " | " | 2,4-dimethylphenyl | " | - | 116-119 |
| 2935 | " | H | " | " | 2,4 dichlorophenyl | " | - | 182-185 |
| 2936 | " | H | " | " | 4-methoxyphenyl | " | - | 177-178 |
| 2937 | " | H | " | " | 2-methylphenyl | " | - | 170-173 |
| 2963 | " | $CH_3$ | " | " | 2,6-diethylphenyl | " | - | 129-132 |
| 2964 | " | " | " | " | 4-phenoxyphenyl | " | - | 151-154 |
| 2965 | " | " | " | " | 3 methylthiophenyl | " | - | 137-138 |
| 2966 | " | " | " | " | 4-benzyloxyphenyl | " | - | 137-139 |
| 2967 | " | " | " | " | 4-dimethylaminophenyl | " | - | 124-127 |
| 3376 | H | $CH_3$ | " | " | phenyl | " | - | 154-155 |
| 3936 | " | " | " | " | 4-fluorophenyl | " | - | 160-161 |
| 3941 | " | " | " | " | 4-nitrophenyl | " | - | 183-185 |
| 3942 | " | " | " | " | 3-nitrophenyl | " | - | 158-161 |
| 3943 | " | " | " | " | 4-benzyloxyphenyl | " | - | 183-185 |
| 3944 | H | $CH_3$ | " | H | 3-methoxyphenyl | S | - | 140-142 |
| 3946 | " | " | " | " | 2-chlorophenyl | " | - | 173-174 |
| 3947 | " | " | | | 3-chloro-2-methylphenyl | " | - | 172-175 |
| 4038 | " | " | " | " | 3-fluorophenyl | " | - | 215-218 |
| 4039 | " | $CH_2CH_2OH$ | " | " | phenyl | " | - | 139-141 |
| 4041 | " | H | " | " | 3-trifluoromethylphenyl | " | - | 205-207 |
| 4042 | " | " | " | " | 4-methoxyphenyl | " | - | 218-220 |

| Comp'd. No. | R | $R_1$ | Y | $R_2$ | $R_3$ | X | Z | m.p.°C |
|---|---|---|---|---|---|---|---|---|
| 4043 | H | H | 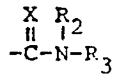 | | 3,4-dichlorophenyl | S | - | 221-223 |
| 4044 | " | $CH_3$ | " | " | 2,5-dimethylphenyl | " | - | 161-164 |
| 4046 | " | H | " | " | phenyl | " | - | 219-221 |
| 4135 | " | $CH_3$ | " | " | 4-trifluoromethylphenyl | " | - | 177-180 |
| 4136 | " | " | " | " | 2-fluorophenyl | " | - | 170-172 |
| 4137 | " | " | " | " | 3-bromophenyl | " | - | 167-169 |
| 4138 | " | " | " | " | 3-methylphenyl | " | - | 166-168 |
| 4179 | " | " | " | " | 3-chlorophenyl | " | - | 162-165 |
| 4180 | " | " | " | " | 3,4-dimethylphenyl | " | - | 155-157 |
| 4181 | " | " | " | " | 3-trifluoromethylphenyl | " | - | 111-115 |
| 4182 | " | " | " | " | 4-methylphenyl | " | - | 168-170 |
| 4183 | " | " | " | " | 3,4-dichlorophenyl | " | - | 182-184 |
| 4184 | " | " | " | " | 2,4-dimethylphenyl | " | - | 165-168 |
| 4229 | $CH_3$ | " | " | " | 4-iodophenyl | " | - | 156-161 |
| 4230 | " | " | " | " | 4-bromophenyl | " | - | 170-172 |
| 4231 | " | " | " | " | 2,4-dichlorophenyl | " | - | 208-210 |
| 4232 | " | " | " | " | 4-cyanophenyl | " | - | 200-203 |
| 4241 | " | " | " | " | 4-methylphenyl | " | - | 138-140 |
| 4242 | " | " | " | " | 3,4-dimethylphenyl | " | - | 132-135 |
| 4243 | " | " | " | " | 3-methylphenyl | " | - | 125-126 |
| 4244 | " | " | " | " | 3,4-dichlorophenyl | " | - | 152-154 |
| 4245 | " | " | " | " | 3-bromophenyl | " | - | 135-138 |
| 4246 | $-C_2H_5$ | $CH_3$ | | H | phenyl | S | - | 118-123 |
| 4247 | " | " | " | " | 3-chlorophenyl | " | - | 82-84 |
| 4248 | " | " | " | " | 4-fluorophenyl | " | - | 147-150 |
| 4254 | $-CH_3$ | " | " | " | 2-fluorophenyl | " | - | 164-165 |
| 4255 | " | " | " | " | 3-trifluoromethylphenyl | " | - | 131-133 |
| 4257 | " | " | " | " | 2-cyanophenyl | " | - | 164-166 |
| 4275 | H | " | " | " | 4-bromophenyl | " | - | 161-163 |
| 4276 | " | " | " | " | 2,4-dichlorophenyl | " | - | 187-189 |
| 4277 | " | " | " | " | 4-cyanophenyl | " | - | 171-173 |
| 4278 | " | " | " | " | 4-iodophenyl | " | - | 163-165 |
| 4587 | " | $CH_3$ | 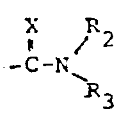 | $CH_3$ | phenyl | " | - | 156-158 |
| 4588 | " | H | " | $CH_3CH_2CH_2$ | phenyl | " | - | 148-151 |
| 4589 | " | $CH_3$ | " | " | phenyl | " | | 167-169 |

| Comp'd. No. | R | $R_1$ | Y | $R_2$ | $R_3$ | X | Z | m.p.°C |
|---|---|---|---|---|---|---|---|---|
| 2559 | $CH_3$ | $CH_3$ | 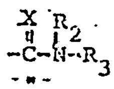 | H | cyclohexyl | S | - | 118-120 |
| 2560 | " | H | " | " | cyclohexyl | " | - | 158-160 |
| 2929 | " | " | " | " | 2-naphthyl | " | - | 183-185 |
| 2934 | " | $CH_3$ | " | $CH_3$ | phenyl | " | - | 98-99 |
| 2938 | " | H | " | H | butyl | " | - | 103-107 |
| 2939 | " | $CH_3$ | " | " | heptyl | " | - | liquid |
| 2940 | " | " | " | " | tert.butyl | " | - | 137-138 |
| 2944 | " | " | " | " | adamantyl | " | - | 171-173 |
| 2945 | " | H | " | " | tert.butyl | " | - | 132-133 |
| 2946 | " | H | " | " | ethyl | " | - | 172-173 |
| 2947 | " | " | " | " | allyl | " | - | 97-101 |
| 4047 | H | " | " | " | isopropyl | " | - | 220-222 |
| 4185 | " | $CH_3$ | " | " | H | " | - | 180-183 |
| 4272 | " | H | " | " | tert.butyl | " | - | 199-203 |
| 4273 | " | " | " | " | allyl | " | - | 210-213 |
| 4274 | " | $CH_3$ | " | " | phenyl | 0 | - | 178-179 |
| 4532 | " | H | 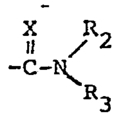 | H | phenyl | 0 | - | 133-134 |
| 4537 | " | " | " | $CH_3$ | 5-(1,1-bischloromethylethyl)-2-thiadiazolyl | 0 | - | 78-79 |
| 4539 | H | $CH_3$ | " | H | 2-fluorophenyl | 0 | - | 207-208 |
| 4540 | " | " | " | " | 2-chlorophenyl | 0 | - | 205-206 |
| 4541 | " | " | " | " | 3-chlorophenyl | " | - | 189-190 |
| 4542 | " | " | " | " | 4-chlorophenyl | " | - | 203-205 |
| 4543 | " | " | " | " | 3-chloro-4-fluorophenyl | " | - | 198-199 |
| 4544 | " | " | " | " | 3-methylphenyl | " | - | 186-188 |
| 4545 | " | " | " | " | 3-methoxyphenyl | " | - | 155-157 |
| 4546 | " | " | " | " | 3-trifluoromethylphenyl | ) | - | 144-147 |
| 4547 | " | " | " | " | 4-isopropylphenyl | " | - | 191-192 |
| 4548 | " | " | " | " | 4-n-butoxyphenyl | " | - | 173-175 |
| 4549 | " | " | " | " | 4-chloro-3-trifluoro-methylphenyl | 0 | - | 181-182 |
| 4550 | " | " | " | " | 2-methylphenyl | " | - | 181-183 |
| 4551 | " | " | " | " | 2,3-dimethylphenyl | 0 | - | 199-201 |
| 4552 | " | " | " | " | 3-ethylphenyl | " | - | 177-179 |
| 4553 | " | " | " | " | 2,6-dichlorophenyl | " | - | 229-231 |

| Comp'd. No. | R | $R_1$ | Y | $R_2$ | $R_3$ | X | Z | m.p.°C |
|---|---|---|---|---|---|---|---|---|
| 4554 | H | $CH_3$ | 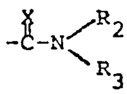 | H | 2-chloro-6-methylphenyl | 0 | - | 197-198 |
| 4555 | H | " | " | " | 4-ethoxyphenyl | " | - | 192-193 |
| 4556 | " | " | " | " | 4-chloro-3-trifluoromethylphenyl | " | - | 206-208 |
| 4557 | " | " | " | " | 3-bromophenyl | " | - | 190-191 |
| 4558 | " | " | " | " | 4-n-butylphenyl | " | - | 178-179 |
| 4559 | " | " | " | " | 4-ethylphenyl | " | - | 200-201 |
| 4560 | " | " | " | " | 2,4-difluorophenyl | " | - | 201-202 |
| 4561 | " | " | " | " | 3-chloro-b-methylphenyl | " | - | 194-195 |
| 4562 | " | " | " | " | 4-ethoxyphenyl | " | - | 184-185 |
| 4563 | " | " | " | " | 4-methoxyphenyl | " | - | 195-196 |
| 4564 | " | " | " | " | 4-fluorophenyl | " | - | 209-210 |
| 4565 | " | " | " | " | 3-chloro-4-methylphenyl | " | - | 202-204 |
| 4566 | " | " | " | " | 3-fluorophenyl | " | - | 174-175 |
| 4567 | " | " | " | " | 2-methyl-3-chlorophenyl | " | - | 289-290 |
| 4568 | " | " | " | " | methyl | " | - | 190-192 |
| 4569 | " | " | " | " | ethyl | " | - | 193-195 |
| 4570 | " | " | " | " | 2-chloroethyl | " | - | 193-194 |
| 4571 | " | " | " | " | isopropyl | " | - | 148-150 |
| 4572 | " | " | " | " | n-butyl | " | - | 166-167 |
| 4573 | " | " | " | " | 5-butyl | " | - | 189-190 |
| 4574 | " | " | " | " | cyclohexyl | " | - | 192-194 |
| 4415 | " | " | 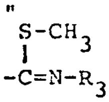 | - | 3-chlorophenyl | - | - | 179-180 |
| 4417 | " | " | " | - | 3-methylphenyl | - | - | 138-140 dec. |
| 4418 | " | " | " | - | 3,4-dichlorophenyl | - | - | 168-172 |
| 4420 | " | " | 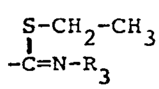 | - | 3-methylphenyl | - | - | 132-135 dec. |
| 4421 | " | " | 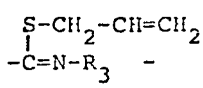 | - | 3-chlorophenyl | - | - | oil |
| 4422 | " | ' | 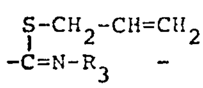 | - | 3-methylphenyl | - | - | oil |
| 4447 | " | " | 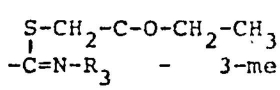 | - | 3-methylphenyl | - | - | oil |

| Comp'd. No. | R | $R_1$ | Y | $R_2$ | $R_3$ | X | Z | m.p.°C |
|---|---|---|---|---|---|---|---|---|
| 4040 | H | $CH_3$ | 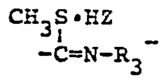 | - | 3-chloro-2-methylphenyl | - | I | 133-135 dec. |
| 4048 | " | " | " | - | 4-fluorophenyl | - | I | 120-122 dec. |
| 4050 | " | H | " | - | phenyl | - | I | 180-182 dec. |
| 4052 | $CH_3$ | $CH_3$ | " | - | phenyl | - | I | 121-126 |
| 4419 | H | " | 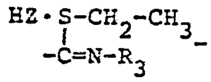 | - | 3-chlorophenyl | - | I | 126-129 |
| 4423 | " | " | " | - | 3,4-dichlorophenyl | - | I | 169-170 |
| 4441 | " | " | " | - | 3-trifluoromethylphenyl | - | I | 164-166 |
| 4442 | " | " | 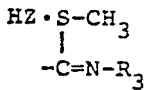 | - | 3-trifluoromethylphenyl | - | I | 177-178 |
| 4448 | " | " | 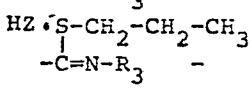 | - | 3-methylphenyl | - | I | oil |
| 4449 | H | " | 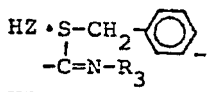 | - | 3-methylphenyl | - | Br | oil |
| 4534 | " | H | 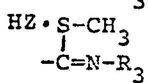 | - | phenyl | - | I | 222-223 |
| 4535 | " | " | 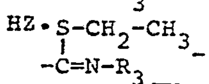 | - | phenyl | - | I | 123-125 |
| 4536 | " | " | 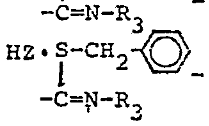 | - | phenyl | - | Br | 181-184 |

Use of the Growth Regulators

By application of an effective amount of the growth regulators, either pre- or post-emergently, various effects on young plants become apparent. These effects may be demonstrated by means of the following illustrative procedures.

Pre-emergent Application

Disposable paper trays about 2 1/2 inches deep which were filled with soil were sprayed with aqueous spray mixtures at a rate of 5 lb. of active chemical per acre of sprayed area, then were seeded with 6 species of plant seeds and were covered with about 1/4 inch of soil. The spray mixtures were prepared by dissolving the proper amount of growth regulator compound in 15 ml of acetone, adding 4 ml of a solvent-emulsifer mixture consisting of 60 wt. percent of a commercial polyoxyethylated vegetable oil emulsifier (96 wt. percent active ingredient, Emulphor EL-719), 20 wt. percent xylene and 20 wt. percent deodorized kerosene, then bringing total volume up to 60 ml by addition of warm water. Twenty-one days after seeding and treatment the plantings were examined and plant injury was rated according to the following schedule.

DEGREE OF EFFECT

0 = no effect
1 = slight effect, plants recovered
2 = moderate effect, injury to 26 to 75 percent
3 = severe effect, injury to 76 to 99 percent of foliage
4 = maximum effect (all plants died)

Post-emergent Application

Several species of plants were grown in potting soil in disposable styrofoam trays and tomatoes were grown in four-inch pots in the greenhouse. Aqueous spray formulations were prepared and the growing plants were sprayed

at a spray volume of 60 gallons per acre and an application rate of 5 lb. per acre. Spray mixtures were prepared in the manner described above. For comparative purposes, plants were also sprayed at 60 gal./acre with a spray mixture containing no growth regulator. Plant injury was again rated according to the schedule disclosed above.

Observations of growth regulator effects in both pre- and post-emergent tests were observed and recorded as follows:

| Effect | Abbreviation in Tables |
|---|---|
| Formative effect on new growth | F |
| Epinasty | E |
| Growth reduction | G |
| Necrosis | N |
| Non-emergence | K |
| Chlorosis | C |

In the table below there are tabulated various observations of pre- and post-emergent herbicidal and growth regulator effects of the compounds disclosed above.

EFFECTS OF COMPOUNDS ON PLANT LIFE

| Compound No. | Pre-emergent Effects | | | | | | Post-emergent Effects | | | | | | Comments |
|---|---|---|---|---|---|---|---|---|---|---|---|---|---|
| | Digitaria sanguinalis | Celosia plumosa | Bromus inermis | Setaria italica | Raphanus sativus | Beta vulgaris | Setaria italica | Medicago sativa | Avena sativa | Raphanus sativus | Beta vulgaris | Lycopersicum esculentum | |
| 2458 | 0 | 0 | F2 | 0 | 0 | F2G1 | 0 | F1 | G1 | F2G1 | F2G2 | F2 | |
| 2556 | 0 | 0 | 0 | 0 | 0 | 0 | N1 | - | 0 | 0 | F1 | 0 | |
| 2557 | F2G1 | F1 | F1 | F2 | 0 | F1 | N3G1 | F1 N2G2 | F2G2 | - | F1 | F2 | |
| 2558 | F1G1 | F1 | F1 | F1G1 | 0 | F1 | F2 | F3G1 | F1 | F2G1 | F2G1 | F3G2E3 | |
| 2924 | F1 | 0 | F1 | F1G1 | 0 | 0 | N1 | F1 | F2 | F1 | F2G2 | F3 | |
| 2925 | 0 | 0 | 0 | 0 | 0 | 0 | N1 | 0 | 0 | 0 | 0 | F2 | |
| 2926 | 0 | 0 | 0 | 0 | 0 | 0 | 0 | 0 | 0 | 0 | F1G2 | N1 | |
| 2927 | 0 | 0 | 0 | 0 | 0 | 0 | 0 | F1 | 0 | 0 | F2G1 | N1F1 | Increase in height of tomatoes |
| 2928 | 0 | 0 | 0 | 0 | 0 | 0 | 0 | G1 | 0 | 0 | 0 | N1 | |
| 2930 | 0 | 0 | 0 | 0 | 0 | 0 | N2 | N1F1 | G1 | 0 | F1G2 | F1 | |
| 2931 | 0 | 0 | 0 | 0 | 0 | 0 | 0 | 0 | 0 | 0 | F2G3 | F1 N1G1 | Increased fruitset and height of tomatoes |
| 2932 | 0 | 0 | 0 | 0 | 0 | 0 | N2 | C1 | 0 | 0 | F3 | N1 | |
| 2933 | 0 | 0 | 0 | 0 | 0 | 0 | 0 | 0 | 0 | 0 | F2G2 | N1 | |
| 2935 | 0 | 0 | 0 | 0 | 0 | 0 | N2G3 | 0 | N1 | N1 | F1 | N1 | |
| 2936 | 0 | 0 | 0 | 0 | 0 | 0 | 0 | 0 | 0 | 0 | F1 | N1 | |
| 2937 | 0 | 0 | 0 | 0 | 0 | 0 | 0 | 0 | 0 | F1 | F2G3 | N1 | |
| 2963 | 0 | 0 | 0 | 0 | 0 | 0 | 0 | 0 | 0 | 0 | F1 | 0 | |
| 2964 | 0 | 0 | 0 | 0 | 0 | 0 | 0 | 0 | 0 | 0 | G2 | 0 | |
| 2965 | 0 | 0 | 0 | 0 | 0 | 0 | 0 | F1 | 0 | 0 | F1 | 0 | |
| 2966 | 0 | 0 | 0 | 0 | 0 | 0 | 0 | 0 | 0 | 0 | F1 | N1 | |
| 2967 | 0 | 0 | 0 | 0 | 0 | 0 | 0 | 0 | 0 | 0 | F1 | 0 | |
| 3376 | F2G1 | 0 | F3G3 | 0 | F2G2 | F3G3 | N2G2 F1 | N1G3 F3 | F1G1 | N2G3 F2 | N3G3 F3 | F2G2 | |
| 3936 | F1 | F1 | F2G1 | F1 | F1G1 | F2G2 | F1G1 | F3G3 | F1 | N4 | N4 | F3C1 | |

EFFECTS OF COMPOUNDS ON PLANT LIFE

| Compound No. | Pre-emergent Effects | | | | | | Post-emergent Effects | | | | | | Comments |
|---|---|---|---|---|---|---|---|---|---|---|---|---|---|
| | Digitaria sanguinalis | Celosia plumosa | Bromus inermis | Setaria italica | Raphanus sativus | Beta vulgaris | Setaria italica | Medicago sativa | Avena sativa | Raphanus sativus | Beta vulgaris | Lycopersicum esculentum | |
| 3941 | 0 | C1 | 0 | 0 | C1 | 0 | F1 | F1 | 0 | N1G1 | N1G1 | F1 | |
| 3942 | 0 | F1 | F2G1 | 0 | 0 | F1 | F2G2 | F3G3 | F1G1 | F1G1 | F1 G2N2 | N4F3 | Increased tomato fruit set |
| 3943 | 0 | 0 | 0 | 0 | 0 | 0 | F1 | 0 | 0 | F1G1 | F1 | F2 | |
| 3944 | F1G1 | F1G1 | K4 | F2G2 | F3G3 | F3G2 | G2F1 | F3G2 | G2F1 | F3G3 | N4 | F3 | |
| 3946 | 0 | N3G3 | G1K2 | 0 | G3 | K4 | N1 | N1G1 | G2 | N4 | N4 | F1 | |
| 3947 | 0 | 0 | F2G1 | 0 | F2G1 | F1 | 0 | F3G1 | 0 | F3G3 | N4 | F3 | |
| 4038 | F2G2 | F2G1 | F3G3 | F1 | F1E1 | F2G2 | F2G1 | F3G2 | F1G1 | F2G2 | F2G1 | F3 | |
| 4039 | 0 | F2 | F2G1 | 0 | 0 | F1 | F1G1 | F3G2 | F1G1 | F2G2 | F3G2 | F3 | |
| 4041 | 0 | F1 | F2 | 0 | F1 | F2G2 | F1 | F3G1 | F1 | F1G1 | F3 | F3 | |
| 4042 | 0 | F2 | F2 | 0 | 0 | F3G2 | F1 | F3G1 | F1 | 0 | F3 | F2 | |
| 4043 | 0 | 0 | F2G1 | 0 | 0 | F1 | F1 | F3 | F1 | F2G1 | F2 | F1 | Promoted tillering, increased fruit set |
| 4044 | 0 | G1 | F3G3 | F1G1 | G3 | F3G3 | F1 | N1 | 0 | G1 | N2 | N2 | |
| 4046 | F3G3 | F2G1 | F3G3 | F3G3 | F3G2 | F3G2 | F2G1 | F3G2 | F2G2 | F3G2 | F3G2 | F3G2 | Increased fruit set, tillering |
| 4135 | 0 | 0 | F1 | 0 | 0 | F1 | F1 | F2G2 | 0 | F3G3 | F3G3 | F3 | Increased axillary growth in tomatoes |
| 4136 | F1 | F1 | F3G2 | F1 | F3G3 | F3G3 | F1G2 | F2G1 | F1 | G2F1 | F2G1 | F2G1 | |
| 4137 | 0 | F2G1 | F2G1 | F1 | F2G2 | F3G3 | F2G2 | F2G1 | F1 | F2G1 | F2G1 | F3E1 | Increased tillering and fruit set |
| 4138 | F1 | F2G2 | F3G1 | F2G2 | F2G2 | F3G2 | F2G2 | F3G3 | F1 | N4 | F3G3 | F3G1 | Increased tillering and fruit set |
| 4179 | 0 | F2G2 | F3G2 | F2G2 | F3G3 | F3G3 | F3G3 | F3G2 | F2G2 | N4 | F3G3 | F3G2 | Increased tillering and fruit set |
| 4180 | F1G1 | 0 | F2G2 | F2G2 | F2G1 | F2G1 | F1 | F1 | 0 | 0 | N1F1 | F2 | Increased fruit set |
| 4181 | 0 | G1 | F1 | 0 | F1 | 0 | F3G2 | E1 F3G3 | F2G2 | N4 | F3G2 | F3E1 | Increased tillering & fruit set |
| 4182 | 0 | 0 | F2 | F1G1 | G3F1 | F1 | 0 | F1 | 0 | N1G1 | F1G1 | F2 | Increased fruit set |
| 4183 | 0 | 0 | F1 | 0 | 0 | 0 | F1 | 0 | 0 | N1G1 | F3G3 | F3 | Increased fruit set |

EFFECTS OF COMPOUNDS ON PLANT LIFE

| Compound No. | Pre-emergent Effects | | | | | | Post-emergent Effects | | | | | | Comments |
|---|---|---|---|---|---|---|---|---|---|---|---|---|---|
| | Digitaria sanguinalis | Celosia plumosa | Bromus inermis | Setaria italica | Raphanus sativus | Beta vulgaris | Setaria italica | Medicago sativa | Avena sativa | Raphanus sativus | Beta vulgaris | Lycopersicum esculentum | |
| 4184 | 0 | 0 | 0 | 0 | 0 | 0 | 0 | 0 | 0 | N1G1 | 0 | 0 | |
| 4229 | 0 | 0 | 0 | 0 | 0 | 0 | 0 | 0 | 0 | 0 | 0 | 0 | |
| 4230 | 0 | 0 | 0 | 0 | 0 | 0 | 0 | 0 | 0 | 0 | 0 | 0 | |
| 4231 | 0 | 0 | 0 | 0 | 0 | 0 | 0 | 0 | 0 | 0 | F1 | F1 | |
| 4232 | 0 | 0 | 0 | 0 | 0 | 0 | 0 | 0 | 0 | 0 | F1 | F1 | |
| 4241 | 0 | 0 | 0 | 0 | 0 | 0 | 0 | 0 | - | 0 | F1 | 0 | |
| 4242 | 0 | 0 | 0 | 0 | 0 | 0 | 0 | 0 | 0 | 0 | F1 | F1 | |
| 4243 | 0 | 0 | 0 | 0 | 0 | 0 | F1 | F1 | - | 0 | F1 | F2G2 | |
| 4244 | 0 | 0 | 0 | 0 | 0 | 0 | 0 | 0 | 0 | 0 | F2 | F1 | |
| 4245 | 0 | 0 | 0 | 0 | 0 | 0 | F1 | F1 | - | 0 | F2G1 | F2G1 | |
| 4254 | 0 | 0 | 0 | 0 | 0 | 0 | 0 | 0 | - | 0 | F1 | F1 | |
| 4255 | 0 | 0 | 0 | 0 | 0 | 0 | 0 | 0 | 0 | 0 | F1 | F1 | |
| 4257 | 0 | 0 | 0 | 0 | 0 | 0 | F1 | 0 | 0 | 0 | F2 | F3 | |
| 4275 | 0 | 0 | 0 | 0 | 0 | F1 | N1 | F2G2 | - | N2G1 | N4 | F3G1 | |
| 4276 | 0 | 0 | 0 | 0 | 0 | 0 | 0 | 0 | 0 | 0 | F2 | F1 | |
| 4277 | 0 | 0 | 0 | 0 | 0 | 0 | F1 | F1 | - | C1 | F2G1 | F1 | |
| 4278 | 0 | 0 | 0 | 0 | 0 | 0 | F2 | F2G2 | - | N2G2 | N4 | F3G1 | |
| 2559 | 0 | 0 | 0 | 0 | 0 | 0 | 0 | 0 | 0 | 0 | 0 | F1 | |
| 2560 | 0 | 0 | 0 | 0 | 0 | 0 | 0 | G1 | 0 | N1 | 0 | N1 | |
| 2929 | 0 | 0 | 0 | 0 | 0 | 0 | N1G1 | 0 | 0 | N1 | F1G1 | N1 | |
| 2934 | 0 | 0 | 0 | 0 | 0 | 0 | N2G1 | F2G2 | G1 | E3G2 | F2G2 | F1 | Increased fruit set |
| 2938 | 0 | 0 | 0 | 0 | 0 | 0 | N2G3 | N1G1 | N1 | N2G1 | F1G2 | N1 | |
| 2939 | 0 | 0 | 0 | 0 | 0 | 0 | 0 | 0 | 0 | 0 | F1 | 0 | |

EFFECTS OF COMPOUNDS ON PLANT LIFE

| Compound No. | Pre-emergent Effects | | | | | | Post-emergent Effects | | | | | | Comments |
|---|---|---|---|---|---|---|---|---|---|---|---|---|---|
| | Digitaria sanguinalis | Celosia plumosa | Bromus inermis | Setaria italica | Raphanus sativus | Beta vulgaris | Setaria italica | Medicago sativa | Avena sativa | Raphanus sativus | Beta vulgaris | Lycopersicum esculentum | |
| 2940 | 0 | K3C1 | 0 | 0 | 0 | 0 | 0 | 0 | 0 | 0 | 0 | 0 | |
| 2944 | 0 | 0 | 0 | 0 | 0 | 0 | N1 | N1 | 0 | 0 | F1 | 0 | |
| 2945 | 0 | 0 | 0 | 0 | 0 | 0 | 0 | 0 | 0 | N1 | F1G2 | N1 | |
| 2946 | 0 | N1 | N1 | C1 | N1 | 0 | F2 C1N2 | N2 | N1 | N1G2 | N1G2 | N1F2 | |
| 2947 | 0 | 0 | 0 | 0 | 0 | 0 | F1 G1N1 | F2G1 | N1G1 | N1 | F2G1 | N1 | |
| 4047 | 0 | F1 | F2 | 0 | 0 | F1 | 0 | N3G3 | - | N2 | N4 | N2 | |
| 4050 | 0 | F1 | F2 | 0 | F1 | F1 | 0 | F2 | 0 | F1G1 | F2 | F3G1 | |
| 4185 | 0 | 0 | 0 | 0 | 0 | 0 | 0 | 0 | 0 | F1 | F2G1 | F2 | |
| 4272 | 0 | F1 | F2 | F2G1 | F1G1 | F1 | 0 | F2G1 | 0 | 0 | F3G1 | F1 | Increased fruit set |
| 4273 | 0 | F1 | F2 | 0 | 0 | 0 | 0 | N1F1 | 0 | 0 | F1 | F1 | |
| 4274 | F3G3 | F3G2 | K4 | F2G2 | F3G2 | F2G2 | F2G2 | F3G3 | - | N2G3 | N4 | F3G2 | |
| 4415 | F1 | F1 | F3G1 | F2G1 | F1G1 | F2 | F1G1 | F3G2 | F1 | F2G1 | F3G2 | F3 | Increased fruit set |
| 4416 | 0 | 0 | 0 | 0 | 0 | F1 | 0 | 0 | 0 | 0 | F1 | F1 | |
| 4417 | F2G2 | F2G2 | F3G1 | G2F1 | G2 | F2G2 | N1G1 | 0 | {F3G2 N2 | F1 | F2G1 | F2 | |
| 4418 | G2F1 | - | G1 | G2F1 | G1 | G1 | N2G2 | {N3G3 F3 | F1 | N1F1 | N4 | F1 | Increased fruit set |
| 4419 | G2F1 | K4 | F3G2 | F3G3 | F2G1 | F3G1 | N2G2 | F3G2 | F1 | F2G1 | N4 | F3 | Increased tillering & fruit set |
| 4420 | F2G1 | F1G1 | F3G1 | F2G2 | F2G2 | F1 | F2G1 | F3G3 | F2 | F2G1 | F3G2 | F3 | " |
| 4421 | F1 | F1 | F2G1 | F2G1 | F2 | F2G1 | N3F2 | F3G2 | F2G1 | F2G1 | F3G2 | F3G1 | " |
| 4422 | F2G1 | F1 | F2 | F2G1 | F2G1 | F2G1 | N3F2 | F3G3 | F2G1 | F2 | N4 | F3 | " |
| 4423 | 0 | 0 | F1 | G1 | 0 | F1 | N1 | F2 | 0 | F1N1 | F2N2 | F3 | Increased fruit set |
| 4441 | 0 | F1G1 | F1 | 0 | F2G1 | 0 | N4 | F3G3 | F1 | N4 | F2G2 | F3 | Increased tillering |
| 4442 | 0 | 0 | 0 | 0 | F2G1 | 0 | 0 | 0 | 0 | 0 | 0 | 0 | |
| 4447 | 0 | - | 0 | 0 | F1 | F1G1 | N4 | N3G3 | F1 | {F1N2 G2 | N4 | F3E3 | Increased tillering |
| 4448 | G1 | N3G3 | F2G1 | F3G2 | F1G1 | G1 | N4 | F3G3 | F2 | F2G2 | F3G3 | F3G1 | Increased fruit set |
| 4449 | 0 | 0 | F1 | F1G1 | G1 | 0 | N3G2 | F3G3 | F1 | F1 | F3G3 | F2 | " |

The use of many of the growth regulator compounds may be demonstrated by treatment of soybeans (*soja max*) to increase the number of seed pods and by treating tomato plants (*Lycopersicum esculentum*) to increase fruit set. In an illustrative experiment, *Soja max* (Evans variety) and *Lycopersicum esculentum* (Tiny Tim variety) were grown in 4-inch pots (one plant per pot) filled with greenhouse potting soil (2 parts good top soil, 1 1/2 parts builders' sand, 1 1/2 parts peat, fertilized with 5 lb. of 12-12-6 fertilizer and 5 lb. of finely ground limestone per cu. yd.). Aqueous spray formulations were prepared and the potted plants were sprayed at a spray volume of 40 gal. per acre and at application rates of 16, 4, 1 and 1/4 oz. per acre. The spray mixtures were prepared by dissolving the proper amount of growth regulator compound in 15 ml. of acetone, adding 2 ml. of a solvent-emulsifier mixture consisting of 60 wt. percent of a commercial polyoxyethylated vegetable oil emulsifier (96 wt. percent active ingredient, Emulphor EL-719); 20 wt. percent xylene and 20 wt. percent deodorized kerosene, then bringing total volume up to 80 ml by addition of a 0.156 wt. percent aqueous solution of liquid non-ionic dispersant (90 wt. percent active trimethylnonyl polyethylene glycol ether, Tergitol TMN-10). Two replicates were sprayed at all application rates. For comparative purposes, plants were also sprayed at 40 gal./acre with water. The number of seed pods and of fruit as percentage of arithmetic mean of the numbers on untreated plants was observed within approximately three weeks after spray treatment and the results are tabulated below. The severity of growth regulatory effect on the plants was estimated on a scale of 0 to 10 and is also recorded in the following table:

GROWTH REGULATING EFFECTS ON TWO SPECIES

| Comp'd. No. | Rate oz/A. | *Soja max* Pod Count ① Percent in Comparison to Untreated Plants | *Soja max* Severity of Growth Regulating Effect ② | *Lycopersicum esculentum* Fruit Count Percent in Comparison to Untreated Plants | *Lycopersicum esculentum* Severity of Growth Regulating Effect ② |
|---|---|---|---|---|---|
| 2458 | 16 | 133 | 3 | 250 | 2 |
| | 4 | 92 | 1 | 162 | 1 |
| | 1 | 88 | 0 | 220 | 0.5 |
| 2556 | 16 | 104 | 0.5 | 132 | 0 |
| | 4 | 100 | 0 | 132 | 0 |
| | 1 | 88 | 0 | 132 | 0 |
| 2557 | 16 | 100 | 2.5 | 235 | 1.5 |
| | 4 | 104 | 0.5 | 191 | 1 |
| | 1 | 108 | 0 | 74 | 0 |
| 2558 | 16 | 98 | 0 | 122 | 3.5 |
| | 4 | 94 | 0 | 141 | 1 |
| | 1 | 101 | 0 | 94 | 0 |
| 2924 | 16 | 96 | 2 | 176 | 1.5 |
| | 4 | 104 | 0.5 | 147 | 0.5 |
| | 1 | 96 | 0 | 162 | 0 |
| 2927 | 16 | 96 | 2 | 103 | 0 |
| | 4 | 100 | 0 | 147 | 0 |
| | 1 | 96 | 0 | 103 | 0 |
| 2932 | 16 | 142 | 3.5 | 176 | 1 |
| | 4 | 113 | 1.5 | 103 | 0 |
| | 1 | 100 | 0 | 118 | 0 |
| 2935 | 16 | 104 | 0 | 103 | 0 |
| | 4 | 96 | 0 | 103 | 0 |
| | 1 | 104 | 0 | 59 | 0 |
| 3376 | 16 | 163 | 7.5 | 212 | 5 |
| | 4 | 154 | 5.5 | 212 | 3 |
| | 1 | 113 | 2.5 | 318 | 2 |
| 3936 | 16 | 146 | 6.5 | 131 | 8.5 |
| | 4 | 131 | 1.5 | 150 | 6 |
| | 1 | 116 | 1 | 122 | 2.5 |
| 3941 | 16 | 98 | 1.5 | 100 | 0 |
| | 4 | 108 | 0 | 0 | 0 |
| | 1 | 101 | 0 | 50 | 0 |
| 3942 | 16 | 123 | 4 | 1000 | 9 |
| | 4 | 123 | 2 | 400 | 3 |
| | 1 | 98 | 1 | 550 | 1 |

GROWTH REGULATING EFFECTS ON TWO SPECIES

| Comp'd. No. | Rate oz/A. | *Soja max* Pod Count ① Percent in Comparison to Untreated Plants | Severity of Growth Regulating Effect ② | *Lycopersicum esculentum* Fruit Count Percent in Comparison to Untreated Plants | Severity of Growth Regulating Effect ② |
|---|---|---|---|---|---|
| 3943 | 16 | 101 | 1.5 | 50 | 0 |
| | 4 | 101 | 0 | 100 | 0 |
| | 1 | 90 | 0 | 150 | 0 |
| 3944 | 16 | 145 | 7 | 1000 | 8 |
| | 4 | 141 | 4.5 | 700 | 5 |
| | 1 | 127 | 1.5 | 550 | 1 |
| 3946 | 16 | 94 | 0.5 | 300 | 0 |
| | 4 | 101 | 0 | 50 | 0 |
| | 1 | 101 | 0 | 100 | 0 |
| 3947 | 16 | 134 | 6.5 | 700 | 7 |
| | 4 | 112 | 2.5 | 400 | 2.5 |
| | 1 | 116 | 1 | 600 | 1.5 |
| 4136 | 16 | 119 | 1 | 150 | 1.5 |
| | 4 | 100 | 0 | 95 | 0.5 |
| | 1 | 100 | 0 | 136 | 0 |
| 4137 | 16 | 81 | 4 | 218 | 7.5 |
| | 4 | 115 | 2.5 | 191 | 5 |
| | 1 | 108 | 1 | 136 | 1.5 |
| 4138 | 16 | 119 | 5.5 | 177 | 5.5 |
| | 4 | 135 | 2.5 | 163 | 3.5 |
| | 1 | 131 | 1.5 | 150 | 1.5 |
| 4179 | 16 | 100 | 7 | 164 | 7 |
| | 4 | 115 | 5.5 | 164 | 4 |
| | 1 | 135 | 2.5 | 109 | 1.5 |
| 4180 | 16 | 104 | 2 | 123 | 1 |
| | 4 | 92 | 0.5 | 150 | 0 |
| | 1 | 92 | 0 | 109 | 0 |
| 4181 | 16 | 112 | 4.5 | 136 | 5.5 |
| | 4 | 115 | 2 | 136 | 1.5 |
| | 1 | 104 | 0.5 | 123 | 0 |
| 4182 | 16 | 119 | 2.5 | 95 | 0.5 |
| | 4 | 112 | 1.5 | 123 | 0 |
| | 1 | 96 | 1 | 123 | 0 |
| 4183 | 16 | 96 | 1 | 68 | 1 |
| | 4 | 104 | 0 | 41 | 1 |
| | 1 | 104 | 0 | 95 | 0 |

GROWTH REGULATING EFFECTS ON TWO SPECIES

| Comp'd. No. | Rate oz/A. | *Soja max* Pod Count (1) Percent in Comparison to Untreated Plants | *Soja max* Severity of Growth Regulating Effect (2) | *Lycopersicum esculentum* Fruit Count Percent in Comparison to Untreated Plants | *Lycopersicum esculentum* Severity of Growth Regulating Effect (2) |
|---|---|---|---|---|---|
| 4184 | 16 | 115 | 0 | 95 | 0 |
| | 4 | 112 | 0 | 109 | 0 |
| | 1 | 100 | 0 | 82 | 0 |
| 4246 | 16 | 97 | 0 | 125 | 0 |
| | 4 | 89 | 0 | 113 | 0 |
| | 1 | 93 | 0 | 125 | 0 |
| 4247 | 16 | 101 | 1 | 213 | 5.5 |
| | 4 | 101 | 0 | 188 | 1.5 |
| | 1 | 93 | 0 | 113 | 0.5 |
| 4254 | 16 | 89 | 1 | 75 | 0 |
| | 4 | 85 | 0 | 125 | 0 |
| | 1 | 97 | 0 | 88 | 0 |
| 4255 | 16 | 109 | 0 | 138 | 1 |
| | 4 | 109 | 0 | 138 | 0 |
| | 1 | 89 | 0 | 88 | 0 |
| 4257 | 16 | 89 | 1.5 | 175 | 3.5 |
| | 4 | 93 | 1 | 238 | 1.5 |
| | 1 | 101 | 0 | 188 | 0 |
| 4275 | 16 | 146 | 3.5 | 213 | 5 |
| | 4 | 105 | 2.5 | 225 | 1.5 |
| | 1 | 105 | 1 | 150 | 0 |
| 4276 | 16 | 122 | 1.5 | 163 | 2 |
| | 4 | 109 | 0.5 | 213 | 1 |
| | 1 | 97 | 0 | 125 | 0 |
| 4277 | 16 | 89 | 3 | 150 | 0.5 |
| | 4 | 89 | 1 | 113 | 0 |
| | 1 | 101 | 0 | 88 | 0 |
| 4278 | 16 | 126 | 3 | 200 | 3.5 |
| | 4 | 126 | 1 | 188 | 1.5 |
| | 1 | 114 | 0 | 150 | 0 |
| 4185 | 16 | 112 | 2 | 68 | 0.5 |
| | 4 | 135 | 1 | 82 | 0 |
| | 1 | 96 | 0 | 54 | 0 |

(1) Untreated Check = 100

(2) Greenhouse rating on scale of 0, no effect to 10, total kill.

The information presented in tabular form herein will enable a worker in the art to make a selection from among the growth regulator compounds of the invention and to make some judgment with regard to application rates, depending upon the effect which is desired. It may be seen, for example, that total kills of some species of vegetation may occur at application rates as high as 5 to 10 lb. per acre, whereas beneficial effects may be observed on living plants at application rates of 1 lb. per acre or less.

The growth regulator compounds are usually applied in combination with inert carriers or diluents, as in aqueous sprays, granules and dust formulations in accordance with established practice in the art. An aqueous spray is usually prepared by mixing a wettable powder or emulsifiable concentrate formulation of a growth regulator with a relatively large amount of water to form a dispersion.

Wettable powders comprise intimate, finely divided mixtures of growth regulator compounds, inert solid carriers and surface agents. The inert solid carrier is usually chosen from among the attapulgite clays, the kaolin clays, the montmorillonite clays, the diatomaceous earths, finely divided silica and purified silicates. Effective surfactants, which have wetting, penetrating and dispersing ability are usually present in a wettable powder formulation in proportions of from 0.5 to about 10 percent by weight. Among the surface active agents commonly used for this purpose are the sulfonated lignins, naphthalenesulfonates and condensed naphthalenesulfonates, alkylbenzenesulfonates, alkyl sulfates and non-ionic surfactants such as products of condensation of ethylene oxide with alkylphenols.

Emulsifiable concentrates of the growth regulator compounds comprise in each instance, a solution of growth regulator compound in a liquid carrier which is a mixture of water-immiscible solvent and surfactants, including

emulsifiers. Useful solvents include aromatic hydrocarbon solvents such as the xylenes, alkylnaphthalenes, petroleum distillates, terpene solvents, ether-alcohols and organic ester solvents. Suitable emulsifiers, dispersing and wetting agents may be selected from the same classes of products which are employed in formulating wettable powders.

In general, the growth regulators are seldom applied without the presence of a carrier or surfactant. However, direct application to plant seeds prior to planting may be accomplished in some instances by mixing powdered solid growth regulator with seed to obtain a substantially uniform coating which is very thin and comprises only one or two percent by weight or less, based on the weight of the seed. In most instances, however, a nonphytotoxic solvent, such as methanol is employed as a carrier to facilitate the uniform distribution of growth regulator on the surface of the seed.

When a compound is to be applied to the soil, as for a pre-emergence application, granular formulations are sometimes more convenient than sprays. A typical granular formation comprises the growth regulator compound dispersed on an inert carrier such as coarsely ground clay, or clay which has been converted to granules by treatment of a rolling bed of the powdered material with a small amount of liquid in a granulating drum. In the usual process for preparing granular formulations, a solution of the active compound is sprayed on the granules while they are being agitated in a suitable mixing apparatus, after which the granules are dried with a current of air during continued agitation.

Claims:

1. A compound having the general structural formula

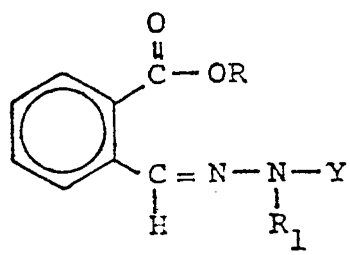 in which Y is 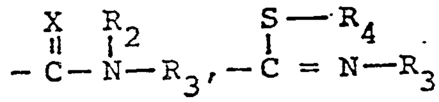

or 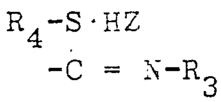 and X is O or S

R is H, $C_1$ to $C_4$ alkyl or an agriculturally acceptable cation

$R_1$ is H or $C_1$ to $C_4$ alkyl or hydroxyalkyl

$R_2$ is H or $C_1$ to $C_3$ alkyl

$R_3$ is H, $C_1$ to $C_8$ alkyl, cycloalkyl or alkenyl, adamantyl, naphthyl, 1,3,4-thiadiazol-2-yl, phenyl or mono- or disubstituted phenyl in which the substituents are selected from $C_1$ to $C_4$ alkyl, $C_1$ to $C_4$ alkoxy, benzyloxy, chloro, bromo, fluoro, iodo, cyano or trifluoromethyl, Z is halogen and $R_4$ is $C_1$ to $C_3$ alkyl, allyl, benzyl or carboethoxymethyl.

2. A compound according to claim 1 in which R and $R_1$ are methyl, Y is 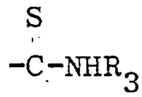, and $R_3$ is phenyl, 3-fluorophenyl, 4-fluorophenyl, 3-chlorophenyl, 4-methoxyphenyl, 2-methylphenyl, 3-methylphenyl, 3-bromophenyl or adamantyl.

3. A compound according to claim 1 in which R is methyl, $R_1$ is H, Y is 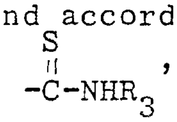, and $R_3$ is 3-chlorophenyl, 3-trifluoromethylphenyl, 2,4-dichlorophenyl, 2-methylphenyl, 2-naphthyl, butyl, ethyl or allyl.

4. A compound according to claim 1 in which R is H, $R_1$ is methyl, Y is 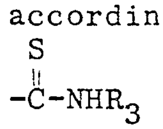 and $R_3$ is phenyl, 4-fluorophenyl,

4-nitrophenyl, 3-nitrophenyl, 4-benzyloxyphenyl, 3-methoxyphenyl, 2-chlorophenyl, 3-chloro-2-methylphenyl, 3-fluorophenyl, 2,5-dimethylphenyl, 4-trifluoromethylphenyl, 2-fluorophenyl, 3-bromophenyl, 3-methylphenyl, 3-chlorophenyl, 3,4-dimethylphenyl, 3-trifluoromethylphenyl, 4-methylphenyl, 3,4-dichlorophenyl, 4-bromophenyl, 4-cyanophenyl or 4-iodophenyl.

5. A compound according to claim 1 in which R is H, $R_1$ is 2-hydroxyethyl, Y is $-\overset{\overset{S}{\|}}{C}-NHR_3$ and $R_3$ is phenyl.

6. A compound according to claim 1 in which R and $R_1$ are H, Y is $-\overset{\overset{S}{\|}}{C}-NHR_3$ and $R_3$ is 3-trifluoromethylphenyl, 4-methoxyphenyl, 3,4-dichlorophenyl, phenyl, isopropyl, <u>tert</u>.butyl or allyl.

7. A compound according to claim 1 in which R and $R_1$ are methyl, Y is $-\overset{\overset{S}{\|}}{C}-NR_2R_3$, $R_2$ is methyl and $R_3$ is phenyl.

8. A compound according to claim 1 in which R is H, $R_1$ is methyl, Y is $-\overset{\overset{O}{\|}}{C}-NHR_3$ and $R_3$ is phenyl.

9. A compound according to claim 1 in which R and $R_1$ are H, Y is $-\overset{CH_3S\cdot HZ}{\overset{|}{C}}=N-R_3$, $R_3$ is phenyl and Z is I.

10. A compound according to claim 1 in which R is H, $R_1$ is methyl, Y is $-\overset{S-CH_3}{\overset{|}{C}}=N-R_3$ and $R_3$ is 3-chlorophenyl, 3-methylphenyl or 3,4-dichlorophenyl.

11. A compound according to claim 1 in which R is H, $R_1$ is methyl, Y is $-\overset{CH_3CH_2-S\cdot HI}{\overset{|}{C}}=N-R_3$ and $R_3$ is 3-chlorophenyl, 3,4-dichlorophenyl or trifluoromethylphenyl.

12. A compound according to claim 1 in which R is H, $R_1$ is methyl, Y is $-\overset{S-CH_2CH_3}{C}=N-R_3$ and $R_3$ is 3-methylphenyl.

13. A compound according to claim 1 in which R is H, $R_1$ is methyl, Y is 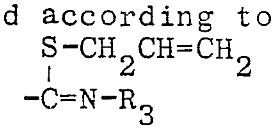 and $R_3$ is 3-chlorophenyl, or methylphenyl.

14. A compound according to claim 1 in which R is H, $R_1$ is methyl, Y is 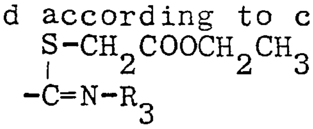 and $R_3$ is 3-methylphenyl.

15. A compound according to claim 1 in which R is H, $R_1$ is methyl, Y is 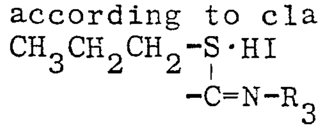 and $R_3$ is 3-methylphenyl.

16. A compound according to claim 1 in which R is H, $R_1$ is methyl, Y is 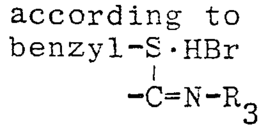 and $R_3$ is 3-methylphenyl.

17. A method of regulating the growth of plants, including combating unwanted vegetation, which comprises applying to the plants, either on seed, the soil or directly on the plants an effective amount of a compound claimed in any of the preceeding claims.

18. A method of manufacturing a plant growth regulating compound having the general structural formula

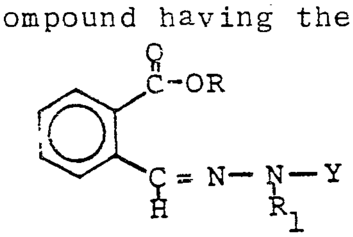

in which X, Y, Z, R, $R_1$, $R_2$, $R_3$ and $R_4$ are as defined in claim 1 which comprises reacting a compound of the formula

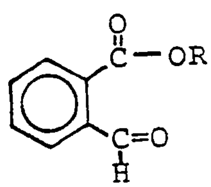

with a compound of the formula 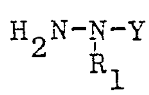 where R, $R_1$ and Y are as defined in claim 1, at ambient room temperature in glacial acetic acid as a dehydrating reagent.

19. A plant growth regulating agricultural composition comprising a compound claimed in any of claims 1 to 16, in combination with an inert carrier and a surface active agent.

European Patent Office

# EUROPEAN SEARCH REPORT

0034010

Application number: EP 81 30 0330

| Category | Citation of document with indication, where appropriate, of relevant passages | Relevant to claim | CLASSIFICATION OF THE APPLICATION (Int. Cl.) |
| --- | --- | --- | --- |
| | **DOCUMENTS CONSIDERED TO BE RELEVANT** | | C 07 C 159/00<br>C 07 C 133/08<br>C 07 D 285/12<br>A 01 N 47/34<br>A 01 N 47/42<br>A 01 N 47/36 |
| X | FR - A - 2 090 478 (EASTMAN KODAK COMP.)<br>* Page 10, table I, comp. 3 *<br>--- | 1 | |
| X | CHEMICAL ABSTRACTS, vol. 64, no. 13, June 20, 1966, ref. 20256e COLUMBUS, OHIO (US)<br>& YAKUGAKU KENKYU 36(10), 319-28 (1965)<br>HYOZO TANIYAMA et al.: "Antimicrobial activity of thiosemicarbazone and related compounds"<br>* The whole abstract *<br>--- | 1 | **TECHNICAL FIELDS SEARCHED (Int. Cl.)** |
| X | CHEMICAL ABSTRACTS, vol. 65, no. 7, September 26, 1966, ref. 11183g COLUMBUS, OHIO (US)<br>& INTERN. CONGR. CHEMOTHERAPY, PROC., 3rd, STUTTGART, 1963,2, 1358-62 (Pub. 1964)<br>B. PRESCOTT et al.: "New thiosemicarbazones as potential chemotherapeutic agents"<br>* The whole abstract *<br>--- | 1 | C 07 C 159/00<br>133/08 |
| X | CHEMICAL ABSTRACTS, vol. 70, no. 17, April 28, 1969, ref. 76696x, page 224<br>COLUMBUS, OHIO (US)<br>& ANN. REP. TAKEDA RES. LAB. 1968, 27, 144-58<br>USUI, YOSHIRO: "Fungicides. XVIII. Antifungal activity of 2-hydrazinothiazole derivatives and thiazolidin-5-ylacetic acid derivatives"<br>* The whole abstract *<br>---<br>./.. | 1 | **CATEGORY OF CITED DOCUMENTS**<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons<br>&: member of the same patent family,<br>corresponding document |

The present search report has been drawn up for all claims

| Place of search | Date of completion of the search | Examiner |
| --- | --- | --- |
| The Hague | 11-05-1981 | PAUWELS |

EPO Form 1503.1 06.78

BAD ORIGINAL

0034010

European Patent Office

EUROPEAN SEARCH REPORT

EP 81 30 0330

-2-

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.) |
|---|---|---|---|
| X | CHEMICAL ABSTRACTS, vol. 75, no. 13, September 27, 1971, ref. 88541 j, page 359<br>COLUMBUS, OHIO (US)<br>& INDIAN J. CHEM. 1971, 9(7), 642-6<br>HUSAIN, SYEDA et al.: "3,4-Disubstituted 5-hydroxy-1,2,4-triazoles derived from 4-substituted semicarbazones"<br>* The whole abstract *<br>---- | 1 | |
| A | CHEMICAL ABSTRACTS, vol. 70, no. 21, May 26, 1969, ref. 96701y page 339<br>COLUMBUS, OHIO (US)<br>& INT. CONGR. CHEMOTHER, PROC., 5th 1967, 1(1) 23-33<br>PRESCOTT B. et al.: "Some new thiosemicarbazones as potential chemotherapeutic agents. Indazolylthiosemicarbazones"<br>---- | 1 | TECHNICAL FIELDS SEARCHED (Int. Cl.) |

EPO Form 1503.2